# EUROPEAN PATENT APPLICATION

(11) **EP 4 640 201 A1**
(43) Date of publication of application: **29.10.2025**
(21) Application number: 25172351.6
(22) Date of filing: 24.04.2025
(51) Int. Cl.: A61K 8/19, A61K 8/24, A61K 8/25, A61K 8/9789, A61K 8/9794, A61Q 11/00, A61K 36/00

(54) **NATURAL TOOTHPASTE, ESPECIALLY FOR SONIC TOOTHBRUSHES**

(30) Priority: 24.04.2024 PL 44840224
(71) Applicant: Uniwersytet Medyczny w Lodzi, 90-419 Lodz (PL); Politechnika Lodzka, 90-924 Lodz (PL)
(72) Inventor: Kula, Zofia, 91-012 Lodz (PL); Smielak, Beata Ewa, 94-234 Lodz (PL); Klimek, Leszek, 95-200 Pabianice (PL)
(74) Representative: Pietruszynska, Elzbieta

(57) **Abstract**

The subject matter of the invention is a toothpaste, especially for sonic toothbrushes, comprising:
- a solvent;
- an abrasive material;
- a thickening agent;
- hydroxyapatite; and
- one of the combinations of ingredients selected from among:
- garlic extract and parsley extract;
- white pearl extract and sea salt; and
- rose extract, viola extract and lavender extract.

## Description

### Field of the Invention

The invention generally relates to the field of oral hygiene. More specifically, it relates to a natural toothpaste designed for the users of sonic toothbrushes.

### Prior Art

Patent specification CN116440036 discloses a toothpaste for teeth whitening and gum protection. The toothpaste disclosed comprises from 0.001% to 5% by weight of thioglycopeptide, from 0.001% to 5% by weight of grapefruit seed extract, from 1% to 20% by weight of hydroxyapatite and from 0.001% to 0.5% by weight of potassium citrate.

Patent specification CN112120987 discloses a toothpaste comprising the following ingredients in parts by weight: 30-75 g of sorbitol, 10-20 g of hydrated silica, 1-2.5 g sodium lauryl sulfate, 0.2-.5 g of Roxburgh rose stock solution, 0.5-1.5 g of sodium phytate, 0.4-1.0 g of cellulose gum, 0.2-0.5 g of carrageenan, 0.1-2 g of wetting agent, 0.1-0.5 g of saccharin sodium, 0.1-0.5 g of sodium benzoate, 0.01-0.1 g of carrageen crispus extract, 0.01-0.1 g of hydroxyapatite, 0.01-0.1 g of xylitol, 0.01-0.1 g of sodium lauroyl sarcosinate, 0.01-0.1 g of sodium methyl ester, 0.01-0.1 g of sodium bicarbonate and 0.01-0.1 g of sodium ascorbyl phosphate.

Patent description CN110731934 discloses a whitening toothpaste that comprises the following ingredients in percentage by weight: 0.01-0.1% of bamboo charcoal, 1-5% of sodium phytate, 1-5% of hydroxyapatite, 1-5% of xylitol, 10-25% of silicon dioxide, 40-70% of sorbitol, 1.8-2.5% sodium lauryl sulfate, 1-5% polyethylene glycol, 0.1-0.5% carrageenan, 0.1-0.5% hydroxymethyl cellulose, 0.1-5% tourmaline, 0.1-2% sucralose, 0.1-1% of licorice extract, 1-3% polyethylene glycol, 0.1-0.3% of trisodium phosphate, 0.6-1.5% of essence, 0.1-0.5% menthol, 0.01-0.2% sodium hyaluronate, 0.1-0.5% 1,2-hexanediol, 0.1-0.5% of caprylyl glycol, 0.1-0.5% p-hydroxyacetophenone, 0.1-2% mica, and the balance of water up to 100%.

Patent specification CN105663015 discloses a toothpaste comprising 0.1-2% by weight of propolis extract, 0.01-5% by weight of lemon extract, 0.01-0.1% by weight of folic acid, 1-8% by weight of hydroxyapatite, 0.01-1% by weight of sodium ascorbyl phosphate and other base materials.

Patent specification WO2014/191009 discloses a toothpaste comprising from 0.5% by weight to 2% by weight of soft propolis extract wherein the soft propolis extract comprises from 85 to 95% by weight of active material.

### Summary of the Invention

### Technical Problem

Despite the availability of a variety of oral prophylaxis products on the market, studies indicate that the oral hygiene of patients of all ages is very poor. Therefore, there is a need to seek increasingly better preparations thanks to which patients will easily take care of their health.

Furthermore, increasingly more people use sonic toothbrushes. The vast majority of commercially available toothpastes are designed for use with a regular manual toothbrush, which does not move as quickly as a sonic toothbrush. The composition of toothpastes for sonic toothbrushes should be characterized by appropriate foaming properties, appropriate abrasiveness and appropriate consistency.

Traditional toothpastes comprising sodium lauryl sulfate (SLS) form aerated foam with large bubbles with low content of active ingredients. Such foam can make it difficult for the active ingredients to reach the tight spaces between teeth and clean them thoroughly. Moreover, commercially available toothpastes for manual toothbrushes often have too high abrasiveness, which can cause micro-damages when used with sonic toothbrushes.

Considering the above and in the light of the presented prior art, the problem to be solved was to provide a toothpaste suitable especially for patients using sonic toothbrushes.

Furthermore, commercially available toothpastes often contain many active ingredients to effectively prevent specific problems in the oral cavity, which may complicate their manufacture and increase the costs of toothpaste production. That is why another problem to be solved was to provide a toothpaste comprising simple combinations of natural easily available active ingredients that do not cause side effects and have a specific activity, such as limiting the formation of bacterial plaque, preventing and alleviating teeth hypersensitivity, eliminating bad breath, treating periodontal diseases, etc.

### Solution of the problem according to the invention

The problem presented above is solved by providing a toothpaste, especially for sonic toothbrushes, having features according to claim 1.

Preferable variants of the toothpaste according to the invention are defined in the dependent claims from 2 to 20.

The subject matter of the invention is a toothpaste, especially for sonic toothbrushes, which comprises:
- a solvent;
- an abrasive material;
- a thickening agent;
- hydroxyapatite; and
- one of the combinations of ingredients selected from among:
   - white pearl extract and sea salt;
   - garlic extract and parsley extract; and
   - rose extract, viola extract and lavender extract.

Preferably, the toothpaste according to the invention further comprises a compound constituting a fluorine source in an amount in the range of 0.3 to 0.9% by weight relative to the total mass of the toothpaste.

Preferably, the toothpaste according to the invention comprises the hydroxyapatite in an amount in the range of 1 to 10% by weight relative to the total mass of the toothpaste.

Preferably, the toothpaste according to the invention comprises the abrasive material selected from the group comprising hydrated silica, silicon dioxide and calcium carbonate, in an amount falling within the range of 15 to 25% by weight relative to the total mass of the toothpaste.

Preferably, the toothpaste according to the invention comprises the thickening agent selected from the group comprising cellulose gum and hydroxyethyl cellulose, in an amount falling within the range of 0.50 to 1.0% by weight relative to the total mass of the toothpaste.

Preferably, the toothpaste according to the invention comprises the solvent in an amount of up to 40.0% by weight relative to the total mass of the toothpaste.

Preferably, the toothpaste according to the invention further comprises a humectant in an amount of up to 35.0% by weight relative to the total mass of the toothpaste.

Preferably, the humectant is selected from the group comprising xylitol, pentylene glycol, glycerine, sorbitol and propylene glycol.

Preferably, the toothpaste according to the invention further comprises a surfactant in an amount of up to 1% by weight relative to the total mass of the toothpaste.

Preferably, the surfactant is selected from the group comprising sodium lauroyl sarcosinate, sodium methyl cocoyl taurate and cocamidopropyl betaine.

Preferably, the toothpaste according to the invention further comprises at least one additive acceptable for use in oral hygiene, selected from the group comprising a preservative, a flavouring agent, a sweetening agent and a colourant.

Preferably, the toothpaste according to the invention comprises the combination of white pearl extract and sea salt.

Preferably, the toothpaste according to the invention comprises the combination of white pearl extract and sea salt in an amount of up to 1% by weight relative to the total mass of the toothpaste.

Preferably, the toothpaste according to the invention, apart from white pearl extract and sea salt, additionally comprises volcanic glass in an amount of up to 2% by weight relative to the total mass of the toothpaste.

Preferably, the toothpaste according to the invention comprises the combination of garlic extract and parsley extract.

Preferably, the toothpaste according to the invention comprises the combination of garlic extract and parsley extract in an amount of up to 1% by weight relative to the total mass of the toothpaste.

Preferably, the toothpaste according to the invention, apart from garlic extract and parsley extract, additionally comprises ribwort plantain extract and/or propolis in an amount of up to 1% by weight relative to the total mass of the toothpaste.

Preferably, the toothpaste according to the invention comprises the combination of rose extract, viola extract and lavender extract.

Preferably, the toothpaste according to the invention comprises the combination of rose extract, viola extract and lavender extract in an amount of up to 1% by weight relative to the total mass of the toothpaste.

Preferably, the toothpaste according to the invention, apart from rose extract, viola extract and lavender extract, additionally comprises camomile extract in an amount of up to 1% by weight relative to the total mass of the toothpaste.

### Advantageous effects of the invention

Thanks to the use of natural abrasive material in the form of hydroxyapatite (HAp) in combination with hydrated silica in the toothpaste according to the invention, low abrasive properties of the toothpaste were obtained with respect to dentin. Sonic toothbrushes as such have very good anti-deposit properties which do not require the use of highly abrasive toothpastes that may cause micro-damages promoting the accumulation of deposits and dental calculus. Thanks to low abrasive properties, the toothpaste according to the invention is also suitable for patients who suffer from teeth hypersensitivity.

Thanks to the use of appropriate proportions of thickening agents, the toothpaste according to the invention has a thick gel-like consistency, which facilitates its use with a sonic toothbrush and improves the distribution of active ingredients.

Furthermore, thanks to the use of a foaming agent having a smaller foaming capacity than sodium lauryl sulfate (SLS), a toothpaste having appropriate foaming properties was obtained.

In addition, the appropriate selection of active ingredients provides toothpastes showing various activities, such as whitening, limiting the formation of bacterial plaque, moisturizing the oral mucosa, eliminating bad breath, eliminating anaerobic bacteria, anti-inflammatory, anticarious, anti-stress and relaxing, among other ones.

### Brief Description of the Figures

Fig. 1 A comparison of the roughness parameter (Ra) of the toothpastes according to the invention with the roughness parameter of comparative commercial toothpastes.
Fig. 2 An exemplary 2D analysis of the surface of a sample brushed using the floral toothpaste (a), the herbal toothpaste (b) and the mineral toothpaste(c) according to the invention.

### Description of embodiments of the invention

Below are discussed individual ingredients of the toothpaste according to the invention.

According to the invention, there is provided a toothpaste suitable for use especially with sonic toothbrushes. The toothpaste according to the invention comprises primarily:
- a solvent;
- an abrasive material;
- a thickening agent;
- hydroxyapatite; and
- one of the combinations of ingredients selected from among:
   - white pearl extract and sea salt;
   - garlic extract and parsley extract; and
   - rose extract, viola extract and lavender extract

The preferable solvent for use in the toothpaste according to the invention is water. Preferably, the solvent is contained in an amount of up to 40.0% by weight, and more preferably in an amount of 30.0% to 40.0% by weight relative to the total mass of the toothpaste.

Any abrasive materials and thickening agents commonly used in oral care products can be used as the abrasive material and the thickening agent.

Preferably, hydrated silica, silicon dioxide or calcium carbonate can be used as the abrasive material. The amount of the abrasive material falls within the range of 15 to 25% by weight relative to the total mass of the toothpaste.

Hydrated silica acts as the abrasive agent and it additionally has the ability to bind the ingredients of the toothpaste and thicken it, thus giving the toothpaste the appropriate structure and viscosity and preventing delamination of the ingredients. Moreover, it can be used in toothpastes with fluorine compounds as it does not react with fluorine.

Despite the fact that the abrasive material such as hydrated silica has thickening properties, the toothpaste according to the invention comprises an additional thickening agent, preferably selected from the group comprising cellulose gum or hydroxyethyl cellulose. The amount of this thickening agent falls within the range of 0.50 to 1.0% by weight relative to the total mass of the toothpaste.

Another ingredient of the toothpaste according to the invention is hydroxyapatite, which has many functions in the toothpaste; namely, it acts as a mild and natural abrasive material, helps to remineralize the enamel, prevents dental calculus build-up, reduces the formation of dental plaque and bacterial biofilm. Its amount falls within the range of 1 to 10% by weight relative to the total mass of the toothpaste. The hydroxyapatite contained in the toothpaste according to the invention is characterized by a particle size in the range of 1.30 µm to 1.9 µm.

Furthermore, as indicated above, the toothpaste according to the invention comprises one of the combinations of active ingredients specified below:
- white pearl extract and sea salt;
- garlic extract and parsley extract; or
- rose extract, viola extract and lavender extract.

In one embodiment, the toothpaste according to the invention comprises the combination of white pearl extract and sea salt. The amount of this combination is up to 1% by weight, preferably up to 0.1%, and more preferably up to 0.05% by weight relative to the total mass of the toothpaste. It is a mineral whitening toothpaste. The mineral ingredients present in the pearl powder, such as calcium, sodium, and magnesium, have a toning and soothing effect, whereas the combination of amino acids and minerals gives a healing effect. White pearl extract absorbs and reflects light and thus it is an excellent protective filter and has whitening properties. The sea salt contained in the toothpaste is a natural abrasive agent which thoroughly removes dental plaque and discolouration accumulated on the enamel surface. Moreover, the sea salt protects teeth from dental caries and calculus, and also protects against gum diseases. During whitening hard tooth tissues, the dentinal tubules become exposed, which leads to hypersensitivity. As a result of using hydroxyapatite in the toothpaste, this process will be blocked by embedding hydroxyapatite deep into the dentinal tubules, which will minimize painful hypersensitivity. In another embodiment, the mineral whitening toothpaste comprises an additional whitening ingredient, such as volcanic glass which contains up to 6% of water in its structure. The volcanic glass is preferably contained in an amount of up to 2% by weight relative to the total mass of the toothpaste. The combination of the above-indicated ingredients resulted in a toothpaste that whitens teeth in a natural way and at the same time limits the formation of bacterial plaque, remineralizes the teeth and moisturizes the oral mucosa.

In the second embodiment, the toothpaste according to the invention comprises the combination of parsley extract and garlic extract. The amount of this combination is up to 1% by weight, preferably up to 0.1% by weight, and more preferably up to 0.05% by weight relative to the total mass of the toothpaste. It is an herbal toothpaste. Parsley and extract thereof contain a very large amount of vitamin C and are one of the richest sources of vitamin A, calcium, magnesium and iron. Due to the large amount of nutrients, parsley shows regenerative, anti-inflammatory and antibacterial effects. An addition of parsley extract effectively eliminates bad breath. Garlic (Allium sativum L.), on the other hand, contains odourless glycoside (alliin), which, under the influence of the alliinase enzyme, breaks down into fructose and allicin, which is a volatile sulphur compound having bactericidal properties. Apart from allicin, garlic and its extract contain other active substances, such as phytosterols, γ-glutamyl peptides, amino acids, vitamins C, PP, B1, B2, A. Additionally, it has anti-inflammatory, antiviral and antifungal properties. In a preferable embodiment of the herbal toothpaste, the toothpaste additionally comprises propolis and/or ribwort plantain extract. These additional ingredients are contained in a total amount of up to 1% by weight, preferably up to 0.1% by weight, and more preferably up to 0.05% by weight relative to the total mass of the toothpaste or one of these ingredients is contained in the above-indicated amount. The function of propolis is to alleviate inflammation of the oral cavity, as well as ailments related to aphthae or thrush. The use of propolis reduces the synthesis of enzymes produced by bacteria causing caries (Streptococcus muntans and Lactobacillus). Propolis also accelerates the healing of wounds and counteracts teeth hypersensitivity. Ribwort plantain, on the other hand, has an anti-inflammatory effect on the oral mucosa, accelerates the healing of wounds related to aphthae or bed sores, moistures, and also contains minerals and vitamins C and K. Therefore, the above herbal toothpaste according to the invention is dedicated for patients struggling with halitosis and gum problems.

Another embodiment of the toothpaste according to the invention is a toothpaste that comprises the combination of rose extract, viola extract and lavender extract. The amount of this combination is up to 1% by weight, preferably up to 0.5% by weight relative to the total mass of the toothpaste. It is a floral toothpaste. Viola herb is a medicinal raw material. It is an important source of many bioactive compounds of plant origin. Viola contains vitamin C, methyl salicylate, mineral salts, flavonoids, anthocyanins, phenolic acids, triterpenoid saponins, hydroxycoumarins, cyclotides, alkaloids, essential oils, tannins and mucilage. Viola has anti-oxidizing, anti-inflammatory, antibacterial, as well as anti-stress properties. Rose extract and lavender extract, on the other hand, have analgesic, antioedematous and anti-inflammatory effects (*inter alia,* they heal gum inflammation) and also reduce gum redness. Moreover, lavender extract has a calming effect and neutralizes the effects of emotional stress and nervous tension. In a preferable embodiment, the toothpaste additionally comprises camomile extract in an amount of up to 1% by weight, more preferably up to 0.1% by weight, and most preferably up to 0.01% by weight relative to the total mass of the toothpaste. The toothpaste comprising floral extracts is designed in particular for patients struggling with periodontal diseases and oral mucosal diseases. In particular, it is recommended for people under severe stress. Some symptoms of stress are visible in the oral cavity, such as ground teeth, ulcers (aphthae), xerostomia, teeth grinding at night or gum problems. The above-indicated combination of floral extracts has stress-reducing, relaxing as well as anti-inflammatory and anticarious effects.

Furthermore, the toothpaste according to the invention may additionally comprise a compound constituting a fluorine source and other additives acceptable for use in oral hygiene.

Any compound commonly used in the field of toothpastes can be included as the compound constituting a fluorine source; namely, sodium monofluorophosphate, sodium fluoride, calcium fluoride, tin fluoride or aluminium fluoride. The compound constituting a fluorine source is contained in an amount in the range of 0.3 to 0.9% by weight relative to the total mass of the toothpaste, which corresponds to the amount of 800 to 1000 ppm (mg/kg of the toothpaste).

In an embodiment, the toothpaste according to the invention further comprises a humectant in an amount of up to 35.0% by weight, preferably from 30.0% to 35.0% by weight relative to the total mass of the toothpaste. Exemplary humectants for use in the toothpaste according to the invention include xylitol, pentylene glycol, glycerine, sorbitol and propylene glycol.

In an embodiment, the toothpaste according to the invention additionally comprises a surfactant in an amount of up to 1.0% by weight, preferably from 0.5% to 1.0% by weight relative to the total mass of the toothpaste. Surfactants contained in toothpastes, as a result of reducing surface tension and cleaning properties, support the action of abrasive compounds. They should also show the ability to form foam having high stability, density and compactness, and should maintain surface activity within a wide pH range. Exemplary surfactants (foaming agents) for use in the toothpaste according to the invention include sodium lauroyl sarcosinate, sodium methyl cocoyl taurate and cocamidopropyl betaine.

In another embodiment, the toothpaste according to the invention further comprises other additives acceptable for use in oral hygiene. Such additives are selected from the group comprising a preservative, a flavouring agent, a sweetening agent and a colourant. Any combination of the above-mentioned agents can be used.

Exemplary preservatives for use in the toothpaste according to the invention include hydroxyacetophenone, phenoxyethanol, methylparaben, ethylparaben and sodium benzoate.

Exemplary flavouring agents for use in the toothpaste according to the invention include limonene (orange fragrance), geraniol (rose flower and geranium flower fragrance), benzyl alcohol (jasmine fragrance), eugenol (carnation fragrance), linalool (lily-of-the-valley fragrance), and citronellol (citrus fragrance).

Exemplary sweetening agents for use in the toothpaste according to the invention include xylitol, sorbitol, sodium saccharin and sucralose.

Exemplary colourants for use in the toothpaste according to the invention include quinoline yellow, phthalocyanine blue, carmine and tartrazine.

The above-specified additives acceptable for use in oral hygiene are contained in the total amount that adds up to 100% by weight. When selecting suitable ingredients and their amounts, it should be taken into account that some ingredients have various functions. For example, sorbitol is a humectant and also a sweetening agent, hydrated silica acts as an abrasive agent and a thickening agent, whereas hydroxyapatite is both an abrasive agent and a remineralizing agent.

### Examples

### Formulas of the toothpastes according to the invention

Below are presented exemplary formulas of the toothpastes according to the invention and their densities. The content of ingredients of natural origin in the below-described toothpastes according to the invention is, respectively: about 98% in the mineral whitening toothpaste and about 97% in the floral and herbal toothpastes.

**Table 1. Formula of whitening mineral toothpaste**

| **Ingredient name** | **Function** | **Content in toothpaste** [%] |
|---|---|---|
| Water | Solvent | 22.291 |
| Xylitol | Sweetening agent, remineralizing agent, humectant | 5.0 |
| Hydroxyacetophenone | Preservative, antioxidant | 0.6 |
| Pentylene glycol | Humectant | 1.5 |
| Glycerine | Humectant | 3.0 |
| Cellulose gum | Thickening agent | 0,8 |
| Sorbitol + water | Humectant, sweetening agent + solvent | 22.56 + 8.4 |
| Volcanic glass | Active ingredient - whitening and abrasive | 1.5 |
| Preparation comprising hydroxyapatite + auxiliary substances (sodium olivoyl glutamate, water, ethylhexylglycerin, caprylyl glycol, phenoxyethanol, propylene glycol) | Abrasive material, active ingredient + auxiliary substances | 5.0 + 7.5 (including 5.0 of water) |
| Sodium monofluorophosphate | Compound constituting a fluorine source | 0.7 |
| Preparation comprising white pearl extract + sea salt + auxiliary substance (glycerine, water, phenoxyethanol) | Active ingredient + active ingredient + auxiliary substances | 0.01 + 0.01 + 0.192 |
| Hydrated silica | Abrasive material, thickening agent | 18 |
| Sodium lauroyl sarcosinate + water | Foaming agent, surfactant + solvent | 0.69 + 1.31 |
| Preparation comprising aroma and limonene | Flavouring agent | 0.5 |
| Preparation comprising calcium and aluminium borosilicate, CI 77891, silica and tin oxide | Colourant | 0.437 |

**Table 2. Formula of herbal toothpaste**

| **Ingredient name** | **Function** | **Content in toothpaste** [%] |
|---|---|---|
| Water | Solvent | 20.009 |
| Xylitol | Sweetening, remineralizing agent, humectant | 5.0 |
| Hydroxyacetophenone | Preservative, antioxidant | 0.6 |
| Pentylene glycol | Humectant | 1.5 |
| Glycerine | Humectant | 3.0 |
| Cellulose gum | Thickening agent | 0.8 |
| Sorbitol + water | Humectant, sweetening agent + solvent | 22.56 + 8.41 |
| Preparation comprising hydroxyapatite and auxiliary substances (sodium olivoyl glutamate, water ethylhexylglycerin, caprylyl glycol, phenoxyethanol, propylene glycol) | Abrasive material, active ingredient + auxiliary substances | 5.0 + 7.5 (including 5.0 of water) |
| Sodium monofluorophosphate | Compound constituting a fluorine source | 0.7 |
| Preparation comprising ribwort plantain extract + auxiliary substances (glycerin+water) | Active antibacterial ingredient + auxiliary substances | 0.01+ 0.096 |
| Preparation comprising garlic extract + auxiliary substances (propylene glycol, water) | Active ingredient + auxiliary substances | 0.01 + 0.1 |
| Preparation comprising parsley extract + auxiliary substances (water, glycerine, sodium benzoate, potassium sorbate) | Active ingredient+ auxiliary substances | 0.003 + 0.1006 |
| Preparation comprising propolis + auxiliary substance (propanediol) | Active ingredient conditioning gums and oral cavity + auxiliary substance | 0.003 + 0.099 |
| Hydrated silica | Abrasive material, thickening agent | 22 |
| Sodium lauroyl sarcosinate + water | Foaming agent, surfactant + solvent | 0.69 + 1.095 |
| Preparation comprising aroma, carvone, menthol and eucalyptus oil | Flavouring ingredient | 0.5 |
| Preparation comprising green colourant (CI 75810) + auxiliary substances (maltodextrin + water) | Colourant+ solvent | 0.005 + 0.1194 |
| Tartrazine (CI 19140) + water | Colourant+ solvent | 0.0009 + 0.0891 |

**Table 3. Formula of floral toothpaste**

| **Ingredient name** | **Function** | **Content in toothpaste** [%] |
|---|---|---|
| Water | Solvent | 20.009 |
| Xylitol | Sweetening, remineralizing agent, humectant | 5.0 |
| Hydroxyacetophenone | Preservative, antioxidant | 0.6 |
| Pentylene glycol | Humectant | 1.5 |
| Glycerine | Humectant | 3.0 |
| Cellulose gum | Thickening agent | 0.8 |
| Sorbitol + water | Humectant, sweetening agent + solvent | 22.56 + 8.44 |
| Preparation comprising hydroxyapatite + auxiliary substances (sodium olivoyl glutamate, ethylhexylglycerin, caprylyl glycol, phenoxyethanol, propylene glycol, water) | Abrasive material, active ingredient + auxiliary substances | 5.0 +7.5 (including 5.0 of water) |
| Sodium monofluorophosphate | Compound constituting a fluorine source | 0.7 |
| Preparation comprising rose extract(ROSA DAMASCENA FLOWER WATER) + auxiliary substances (sodium benzoate, gluconolactone, calcium gluconate) | Active ingredient + auxiliary substances | 0.1 + 0.00172 |
| Preparation comprising camomile extract + auxiliary substances (glycerine, sodium benzoate, potassium sorbate, water) | Active ingredient + auxiliary substances | 0.005 + 0.1206 |
| Preparation comprising lavender extract (LAVANDULA ANGUSTIFOLIA FLOWER EXTRACT) and auxiliary substances (glycerine, water, sodium benzoate, potassium sorbate) | Active ingredient + auxiliary substances | 0.005+ 0.1206 |
| Preparation comprising viola extract(VIOLA TRICOLOR EXTRACT) and auxiliary substances (glycerine, sodium benzoate, potassium sorbate, water) | Active ingredient + auxiliary substances | 0.003 + 0.1206 |
| Hydrated silica | Abrasive material, thickening agent | 22 |
| Sodium lauroyl sarcosinate + water | Foaming agent, surfactant + solvent | 0.69 + 1.095 |
| Preparation comprising aroma, benzyl alcohol, CITRONELLOL GERANIOL | Flavouring agent | 0.6 |
| CI 16035 + water | Colourant + solvent | 0.003 + 0.02178 |
| CI 19140 + water | Colourant + solvent | 0.0007 + 0.004 |

The densities of the above-indicated toothpastes are as follows:
- the mineral whitening toothpaste - 1.33 g/cm³;
- the herbal toothpaste - 1.3 g/cm³;
- the floral toothpaste - 1.36 g/cm³;

Whereas exemplary commercial toothpastes have, for example, the following densities: 1.15 g/cm³ (Madonis toothpaste) or 1.21 g/cm³ (Colgate Prevent toothpaste).

### Tests of abrasive properties

Tests of the abrasive properties of the toothpastes according to the invention and comparative toothpastes available on the market were conducted in accordance with ISO standard PN-EN 11-609 concerning dentifrices. One of the recommended methods of determining the abrasive properties of toothpastes is a method using a profilometer. This method usually uses the parameter of roughness Ra, which is the arithmetic mean of absolute values.

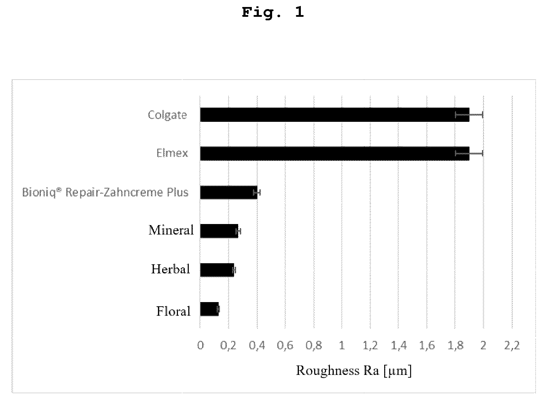

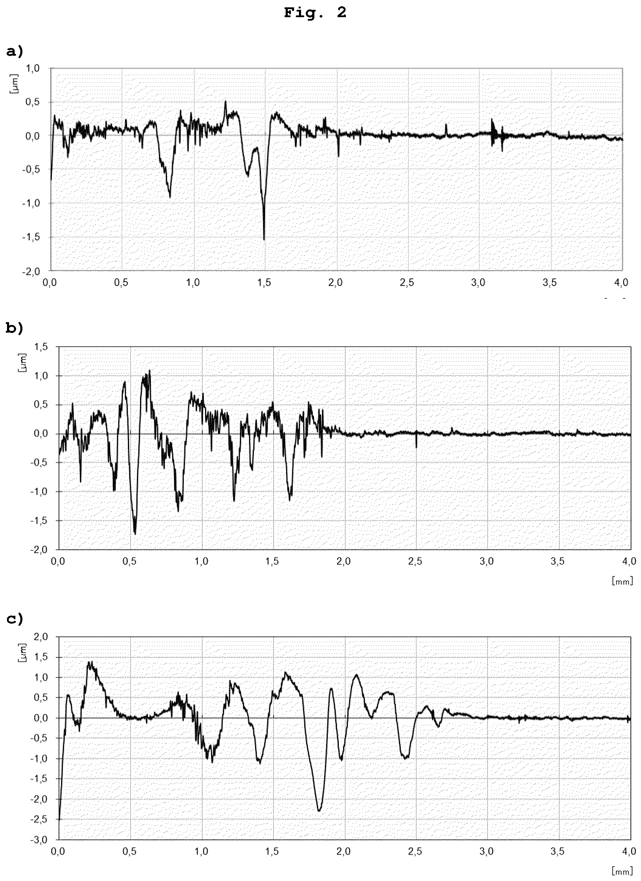

In this in vitro test, the influence of the concentration of abrasive particles on toothpaste abrasiveness was tested by means of a profilometer. PMMA plates (imitating tooth tissues) were tested in a sonic toothbrush simulator with distilled water and experimental toothpastes. The results presented in Fig. 1 show that the abrasiveness of the toothpastes according to the invention is lower than that of the commercial toothpastes that were the comparative toothpastes. Fig. 2 presents an exemplary profile of samples brushed with the toothpastes according to the invention; namely, Fig. 2 a) concerns the floral toothpaste, Fig. 2 b) - the herbal toothpaste, and Fig. 2 c) - the mineral whitening toothpaste.

### Evaluation of toothpaste properties

Sixteen volunteers (8 women and 8 men) aged from 25 to 70 participated in the evaluation of the properties of the toothpastes described in Tables 1-3. Each participant filled in a questionnaire with questions concerning the properties of the toothpastes and their effects. The declared effect of a product is considered to be confirmed when the number of positive responses constitutes at least 50% of the total number of responses obtained from the respondents.

The obtained results of the questionnaires show that all or the majority of the respondents considered the toothpaste colour and flavour and ease of spreading it as definitely suitable or suitable for them (100%, 98% and 97%, respectively).

In the opinion of 94% of the respondents, the toothpastes according to the invention remove dental plaque and prevent calculus build-up.

95% of the respondents indicated that the toothpastes effectively cleaned without causing micro-damages of the enamel.

Furthermore, in the opinion of 65% of the respondents, the toothpastes strengthen the gums' resistance to irritation and leave a refreshing effect.

## Claims

1. A toothpaste, especially for sonic toothbrushes, **characterized in that** it comprises:
- a solvent;
- an abrasive material;
- a thickening agent;
- hydroxyapatite; and
- one of the combinations of ingredients selected from among:
- garlic extract and parsley extract;
- white pearl extract and sea salt; and
- rose extract, viola extract and lavender extract.

2. The toothpaste according to claim 1, **characterized in that** the toothpaste further comprises a compound constituting a fluorine source in an amount in the range of 0.3 to 0.9% by weight relative to the total mass of the toothpaste.

3. The toothpaste according to claim 1 or 2, **characterized in that** the toothpaste comprises the hydroxyapatite in an amount in the range of 1 to 10% by weight relative to the total mass of the toothpaste.

4. The toothpaste according to any one of claims from 1 to 3, **characterized in that** the toothpaste comprises the abrasive material selected from the group comprising hydrated silica, silicon dioxide and calcium carbonate, in an amount falling within the range of 15 to 25% by weight relative to the total mass of the toothpaste.

5. The toothpaste according to any one of claims from 1 to 4, **characterized in that** the toothpaste comprises the thickening agent selected from the group comprising cellulose gum and hydroxyethyl cellulose, in an amount falling within the range of 0.50 to 1.0% by weight relative to the total mass of the toothpaste.

6. The toothpaste according to any one of claims from 1 to 5, **characterized in that** the toothpaste comprises the solvent in an amount of up to 40.0% by weight relative to the total mass of the toothpaste.

7. The toothpaste according to any one of claims from 1 to 6, **characterized in that** the toothpaste further comprises a humectant in an amount of up to 35.0% by weight relative to the total mass of the toothpaste, wherein preferably the humectant is selected from the group comprising xylitol, pentylene glycol, glycerine, sorbitol and propylene glycol.

8. The toothpaste according to any one of claims from 1 to 7, **characterized in that** the toothpaste further comprises a surfactant in an amount of up to 1% by weight relative to the total mass of the toothpaste, wherein preferably the surfactant is selected from the group comprising sodium lauroyl sarcosinate, sodium methyl cocoyl taurate and cocamidopropyl betaine.

9. The toothpaste according to any one of claims from 1 to 8, **characterized in that** the toothpaste further comprises at least one additive acceptable for use in oral hygiene, selected from the group comprising a preservative, a flavouring agent, a sweetening agent and a colourant.

10. The toothpaste according to any one of claims from 1 to 9, **characterized in that** the toothpaste comprises the combination of white pearl extract and sea salt, wherein preferably the combination of white pearl extract and sea salt is contained in an amount of up to 1% by weight relative to the total mass of the toothpaste.

11. The toothpaste according to claim 10, **characterized in that** the toothpaste additionally comprises volcanic glass in an amount of up to 2% by weight relative to the total mass of the toothpaste.

12. The toothpaste according to any one of claims from 1 to 9, **characterized in that** the toothpaste comprises the combination of garlic extract and parsley extract, wherein preferably the combination of garlic extract and parsley extract is contained in an amount of up to 1% by weight relative to the total mass of the toothpaste.

13. The toothpaste according to claim 12, **characterized in that** the toothpaste additionally comprises ribwort plantain extract and/or propolis in an amount of up to 1% by weight relative to the total mass of the toothpaste.

14. The toothpaste according to any one of claims from 1 to 9, **characterized in that** the toothpaste comprises the combination of rose extract, viola extract and lavender extract, wherein preferably the combination of rose extract, viola extract and lavender extract is contained in an amount of up to 1% by weight relative to the total mass of the toothpaste.

15. The toothpaste according to claim 14, **characterized in that** the toothpaste additionally comprises camomile extract in an amount of up to 1% by weight relative to the total mass of the toothpaste.
